# EUROPEAN PATENT SPECIFICATION

(11) **EP 0 679 401 B1**
(45) Date of publication and mention of the grant of the patent: **20.08.2003**
(21) Application number: 95900913.5
(22) Date of filing: 17.11.1994
(51) Int. Cl.: A61K 33/06, A61K 33/10, A61K 31/70, A61K 31/19, A61K 47/02, A61K 9/08

(54) **COMPOSITION FOR CURING LESIONED TISSUE, PROCESS FOR PRODUCING THE SAME, AND USE THEREOF**
ZUSAMMENSETZUNG ZUM HEILEN VON VERLETZTEM GEWEBE, VERFAHREN FÜR IHRE HERSTELLUNG UND IHRE VERWENDUNG
COMPOSITION DESTINEE AU TRAITEMENT DE TISSUS PRESENTANT DES LESIONS, PROCEDE DE PRODUCTION ET LEUR UTILISATION

(30) Priority: 17.11.1993 JP 28832393; 22.07.1994 JP 19221994
(43) Date of publication of application: 02.11.1995
(73) Proprietor: LEQUIO PHARMA CO., LTD., Naha-shi, Okinawa (JP); Mitsubishi Pharma Corporation, Osaka-shi, Osaka 541-0046 (JP)
(72) Inventor: OKU, Kinuko, Naha-shi Okinawa 900 (JP)
(74) Representative: Gille Hrabal Struck Neidlein Prop Roos
(86) International application number: JP9401942
(87) International publication number: WO95014480

(56) References cited:
- EP-A- 0 342 524
- WO-A-94/06443
- JP-A- 4 225 920
- US-A- 4 395 398
- US-A- 4 626 433

## Description

### TECHNICAL BACKGROUND

The present invention relates to a method for the preparation of a composition for curing affected abnormal tissues, and more particularly, for curing affected abnormal tissues caused for example by piles or hemorrhoids, which composition is stable during storage for a long period of time.

### BACKGROUND ART

Injection therapy of internal piles or hemorrhoids has a history of more than 100 years. The early injections used for such therapy were composed of a ferrous sulfate solution, phenol in olive oil, and the like. Thereafter, there were used alcohol, quinine hydrochloride, mercuric chloride, urethane, ergot and the like. In the past thirty years, a phenol solution in vegetable oil and an alum solution have been used most frequently for curing the internal piles or hemorrhoids.

These compositions, however, cannot be said to satisfactorily solve all or most of such problems for curing internal piles or hemorrhoids for reasons as will be described hereinafter. It is noted that the injection therapy for sclerosing the internal piles or hemorrhoids requires that the injectable preparations reduce inflammation, stop bleeding, inhibit the growth of bacteria, bring about well-formed thrombosis and complete fibrosis of hemorrhoidal blood vessels without leaving any hard nodules or causing necrosis. The injectable preparations so far developed for curing the internal piles or hemorrhoids by sclerosing the affected abnormal tissues of the digestive system, however, do not satisfy any or a majority of the requirements as described immediately hereinabove. It is also noted as difficult to determine the appropriate concentration and quantity of the injectable preparations used at clinical needs, as well as a procedure to sclerose the internal piles or hemorrhoids without causing necrosis. It is further required that the methods of injection therapy and administration be improved so as to expand the injected area because the composition be deposited only in the submucosa when injected.

As a composition for curing internal piles or hemorrhoids, there is proposed an injectable agent that comprises tannin, alum, sodium citrate, dextran, glycerin and trichlorobutyl alcohol (Journal of Traditional Chinese Medicine: vol. 1, No. 2, pp. 116-120 (1981)). Alum has a chemical nomenclature as aluminum potassium sulfate and it is a colorless and transparent substance of an octahedral crystal. It has locally astringent, hemostatic and antiseptic properties. Alum is not absorbed through the gastroenteric mucosa when administered internally and exerts merely a local action against the mucosa. On the other hand, tannic acid is usually obtained from nutgall or gall and may be a yellow-white or pale brown, amorphous and powdery substance or a small-leaf-shaped or spongy bulky substance. Tannic acid can cause a protein to coagulate in an acidic or neutral solution and has astringent, antiseptic and anti-bacterial properties.

It is further noted that aluminum potassium sulfate is not allowed to be practically used in combination with tannic acid or tannin agents (Pharmacopedia Japonica, The 12th Revision (1991); pp. D-1019-1021). In addition, it is also known that tannic acid forms a less soluble salt with basic organic chemical substances and a non-soluble or sparingly soluble compound with an iron agent or the like, thereby losing its effects.

The injectable preparations as described immediately hereinabove contain trichlorobutyl alcohol so that there is the possibility that it presents the problems as will be described hereinafter. It is known that trichlorobutyl alcohol is a compound in which carbon atoms and chlorine atoms are combined with each other and it can oxidize tannic acid due to the formation of a radical upon the absorption of light, thereby causing tannic acid to turn into precipitated material having a brown or black color. Further, trichlorobutyl alcohol has the property that the linkage between the carbon and chlorine atoms is broken upon application of high energy such as heat or light, turning into unstable substances. It is thus said to be inappropriate to use trichlorobutyl alcohol as a component of a medicine due to such property because there is the highly probable risk that it causes precipitates in the injectable preparations during storage. In addition, the use of such a compound is less convenient in handling and storing because the greatest possible care is required for storing the injectable preparations containing it.

Further, Japanese Patent Unexamined Publication Kokai No. 4-225,920 discloses an agent for hardening or sclerosing the affected abnormal tissues of the digestive system such as, for example, esophagophlebangioma, piles or hemorrhoids, rectal prolapse, prolapse of the rectal mucosa and the affected uplift tissues of the large intestine and the rectum. The sclerosing agent consists of a composition comprising tannic acid, aluminum potassium sulfate and a stabilizing agent including an extract of a medicinal plant, containing a phenol, a flavone or a flavonoid, a catechin or a polycarboxylic acid. As this composition contains the extract of the medicinal plant as the stabilizing agent, however, it suffers from the disadvantage in terms of formulating preparations that it is extremely difficult to provide injectable preparations with a constant formulation and stable quality as well as that there is still the possibility that the stabilizing agent may be contaminated with minute amounts of unidentified substances even if it would be extracted and purified elaborately through plural steps. It has further been found that such a composition comprising tannic acid and aluminum potassium sulfate may cause coloring or, in some cases, precipitating, even if it contains the extract of the plant as a stabilizing agent, when stored for a long period of time in a liquid state. This also may cause a problem when it is applied as an injectable agent.

In addition, PCT International Publication No. WO94/06443 (International Publication Date: March 31, 1994) discloses a composition for curing affected abnormal tissues of the digestive system as an injectable preparation, which comprises a composition containing a water-soluble aluminum compound in a concentration ranging from 0.01 mole to 0.5 mole, tannic acid at a rate of 0.5% to 25.0% with respect to the amount of the water-soluble aluminum compound, sodium hydrogen sulfite and a polyvalent alcohol or a saccharide; in which the pH of said composition is adjusted so as to become in the range of from pH1.5 to pH3.5. The components of the compositions disclosed in this document are the same as those of the ones to which the present invention is directed. This document, however, discloses a method for the preparation of the composition for curing the affected abnormal tissues of the digestive system as well as the compositions consisting of the same components as those of the present invention. The published method comprises formulating all the components of the composition in a simultaneous way or in a random way. More specifically, the method for the preparation of the hardening or sclerosing agent disclosed in the prior art document comprises placing all the components in a test tube, pouring a predetermined amount of distilled water for injection use with stirring in the test tube, and dissolving them to give an aqueous solution. The resulting aqueous solution is then filled in a container such as a glass bottle; followed by removal of oxygen dissolved in the solution and replenishment of air with nitrogen gas. Thereafter, the solution is sterilized with high-pressure steam and then stored in a cold and dark place. It has been found, however, that the general method for the formulation of the composition as disclosed in the prior art document suffers from the extreme difficulty in preparing a stable composition with the constant formulation and that this presents the problem in the actual preparation of the compositions as an injectable agent.

Aluminum potassium sulfate, which may also be called alum and is used as one component for the composition according to the present invention, has been applied as a hemostatic agent. As described hereinabove, however, a combination of aluminum potassium sulfate with a tannic agent is prohibited to be used as medicine as set forth in Pharmacopedia Japonica. Nevertheless, various attempts have been made to use aluminum potassium sulfate in combination with the tannic agent as medicine; however, aluminum potassium sulfate is very unstable when it is mixed with the tannic agent as it is because it may become colored or precipitate. Hence, the use of such an unstable composition as injectable preparation is inappropriate even in terms of safety.

Further, aluminum potassium sulfate usually contains a small amount of iron because it is prepared from bauxite as a raw material. Pharmacopedia Japonica regulates the acceptable contents of iron derivatives to be 20 ppm or less when aluminum potassium sulfate is employed as a raw material for medicine.

In addition, aluminum potassium sulfate may decompose into aluminum ions such as Al³⁺, Al(OH)²⁺, Al(OH)₂⁺, and the like upon dissociation in a solution and react with hydroxyl ion, OH⁻, to form aluminum hydroxide, Al(OH)₃, which precipitates in a solution. If such precipitates are formed in the solution, it is inappropriate to employ it as a composition for injectable preparations and it cannot be applied for treatment.

On the other hand, tannic acid is oxidized to form an oxide compound such as a quinone compound upon exposure to oxygen existing in air or dissolved in a solution to be used, thereby causing precipitates when the solution is used for injectable preparations. If such insoluble products precipitate in the injectable preparations, the resulting injection becomes as a matter of course inappropriate as a medicine and cannot be administered.

Further, tannic acid is oxidized forming precipitates, particularly when iron ions are present in the solution. Hence, if tannic acid exists with aluminum potassium sulfate in the solution, the iron ions present in the aluminum potassium sulfate contained in the solution are reacted directly with tannic acid, leading to the formation of precipitates, and the oxidation of tannic acid is accelerated.

In addition, tannic acid is allowed to react directly with aluminum ions, causing to form precipitates, if the aluminum ions are present in the solution, and it is likely to undergo oxidation.

Accordingly, a composition containing aluminum potassium sulfate and tannic acid cannot be prepared in a constant and stable manner without causing any precipitates simply by formulating all the components in a simultaneous or random way.

As the result of a detailed review on the order and conditions of formulating the components in preparing the preparations for injection, it has been found by the present inventor that injectable preparations for curing affected abnormal tissues having a constant and stable composition can be prepared by arranging the order of formulation of its components and the concentrations thereof. Thus, the present invention has been completed from this finding.

### DISCLOSURE OF INVENTION

Therefore, the present invention can solve the problems involved in conventional compositions for curing the affected abnormal tissues and the present invention has the object to provide a method for the production of the injectable preparations having a constant and stable formulation, for application in order to cure piles or hemorrhoids, affected uplift tissues of the digestive tract, hepatic tumor, and the like.

The composition for curing the affected abnormal tissues in accordance with the method of the present invention may comprise substantially a water-soluble aluminum compound, tannic acid, citric acid or a salt thereof, and sodium hydrogen sulfite. The pH range of the composition is adjusted to be from pH1.0 to pH3.5.

Alternatively, the composition for curing the affected abnormal tissues according to the method of the present invention may further comprise the polyvalent alcohol and/or saccharide in addition to the formulation consisting of the water-soluble aluminum compound, citric acid or a salt thereof, tannic acid and sodium hydrogen sulfite, the composition being adjusted, as needed, to a pH ranging from pH1.0 to pH3.5.

As the water-soluble aluminum compound to be employed for the method for the preparation of the composition for curing the affected abnormal tissues in accordance with the present invention, there may be mentioned, for example, aluminum chloride, aluminum sulfate, aluminum carbonate, aluminum acetate, aluminum nitrate, aluminum lactate, aluminum tartrate, aluminum salicylate, aluminum sodium sulfate, aluminum potassium sulfate, aluminum cesium sulfate, and ammonium aluminum sulfate. The water-soluble aluminum compounds may be applied singly or in combination of two or more.

The water-soluble aluminum compound may be formulated in concentrations ranging usually from 0.01 to 0.5 mole, preferably from 0.03 to 0.3 mole. It is preferred that the water-soluble aluminum compound is contained so as to amount to from approximately 1% to 10%, preferably from approximately 2% to 5%, as the effective concentrations in the injectable preparations

As tannic acid to be employed for the method for the preparation of the curing injectable solution according to the present invention, there may be employed one derived from a variety of plants. The most preferred one is tannic acid derived from gallnut. Tannic acid may be contained at a rate ranging usually from 0.01% to 2.0%, preferably from 0.05% to 1.5%, as the effective concentrations in the injectable preparations. The tannic acid may be added in a concentration beyond the above range by adjusting the amounts of the other components, particularly sodium hydrogen sulfite; however, in this case, care should be paid in preparing and storing the compositions because the resulting compositions are likely to cause coloring or precipitating or depositing during storage for a long period of time. In this sense, such compositions are inconvenient in handling.

As described hereinabove, aluminum potassium sulfate may be decomposed to form aluminum ions such as Al³⁺, Al(OH)²⁺, Al(OH₂)₂⁺ and the like upon dissociation in the solution and react with hydroxyl ion, OH⁻, to form aluminum hydroxide, Al(OH)₃. If such aluminum ions are present in the solution, tannic acid is reacted directly with them, whereby the solution is likely to cause precipitating.

The method of the present invention uses citric acid as a compound having the action of trapping minute amounts of metal ions present in the solution. This may include citric acid or a salt thereof such as sodium citrate. The amount of citric acid or its salt may be in the range of from approximately 10% to 80%, preferably from approximately 20% to 50%, with respect to the amount of the water-soluble aluminum compound, although the amount of citric acid or its salt may be varied with the kind or the amount of the water-soluble aluminum compound.

In preparing the compositions for curing the affected abnormal tissues in accordance with the present invention, citric acid or its salt such as sodium citrate is added to a solution containing the water-soluble aluminum compound before formulating tannic acid with the water-soluble aluminum compound, in order to avoid the direct reaction of the tannic acid with the water-soluble aluminum compound and to cause no precipitating. Once citric acid or its salt is added to the solution containing the water-soluble aluminum compound, it reacts with the aluminum ions present in the solution, resulting in the formation of complex aluminum ions and trapping the metal ions from the solution to which tannic acid is to be added, the metal ions being reactable with the tannic acid and as a result being likely to cause forming precipitates. To the solution treated in the manner as described hereinabove is added tannic acid to thereby yield a solution in which the tannic acid is not reacted directly with the metal ions, such as aluminum ions, derived from the water-soluble aluminum compound and in which no precipitates are formed. Theoretically, for example, if 0.65 molecule of the aluminum ion is present with respect to one molecule of sodium citrate ion and the former is to be linked to the latter, no free aluminum ion is present in the solution. Practically, however, as the sodium citrate ion exists in a neutral state or in the form of a citric acid dihydrogen ion in the range of such a strong acid as the composition prepared according to the present invention, such amounts are insufficient to trap the aluminum ions in the 1.65-fold concentrations as complex ions. Hence, in this case, it is preferred to select a solution having the composition so as to allow free aluminum ions to exist in a range that does not exceed the solubility product constant of aluminum tannate.

Further, tannic acid is unstable against light or air in a solid state and it is less stable in the solution from the point of view of a diffusion velocity between the reactive substances. For the purpose to prevent oxidation of tannic acid, there is used as an antioxidant sodium hydrogen sulfite. The amount of sodium hydrogen sulfite to be used as the antioxidant in accordance with the present invention may be usually from approximately 50% to 200%, preferably from approximately 70% to 150%, with respect to the amount of the tannic acid. If the sodium hydrogen sulfite would be contained in a concentration beyond the above range, the resulting injectable preparations cannot accomplish the desired effects sought to be achieved by the present invention.

In order to make the composition prepared according to the present invention more stable, there may be added as needed a polyvalent alcohol and/or a saccharide, which are or is conventionally employed for curable compositions. As the polyvalent alcohol and/or the saccharide to be employed for the present invention, there may be mentioned glycerin, glucose, fructose, xylitol, mannose, mannitol, galactose, dextran and the like. Particularly, dextran has the action of raising the viscosity of the resulting injectable preparations. The polyvalent alcohol and/or saccharide may be employed singly or in combination of two or more. Further, the amount of the polyvalent alcohol and/or saccharide is not. limited to a particular one as long as the polyvalent alcohol and/or saccharide may be contained at such a rate so as to raise the osmosis of the resulting injectable preparations by approximately three times to fifteen times, preferably from approximately four times to eight times, that of physiological saline. In addition, the polyvalent alcohol and/or saccharide are or is preferably contained in the range of from approximately 3% to 20%, preferably from approximately 5% to 15%, as the effective concentration in the injectable preparations.

Further, the compositions according to the present invention may contain another component which hitherto has been conventionally employed as an ingredient for compositions, such as an agent for increasing viscosity of a solution, e.g. dextran or the like, or an antiseptic agent, e.g. phenol, benzyl alcohol, benzalkonium chloride, p-aminobenzoic acid ester or the like. These components may optionally be employed within the range that does not substantially affect the effects of the compositions adversely.

In accordance with the present invention, it is of significance in terms of storage of the resulting preparations that the liquid property of the injectable preparations containing the composition is adjusted to be from pH1.0 to pH3.5, preferably from pH2.0 to pH3.0.

The compositions prepared by means of the method according to the present invention, have liquid properties within the above-defined range without special adjustment and, in this case, it is not particularly required to adjust the liquid properties of the resulting injectable preparations after or before the preparation of the compositions. It is also possible to adjust the liquid properties of the compositions, as needed, to the optimal level with a pharmacologically nontoxic acid or alkali, which are conventionally used for the formulation of the injectable preparations. Such agents may include, for example, a mineral acid such as hydrochloric acid or sulfuric acid, an organic acid such as citric acid, sodium hydrogen carbonate, sodium hydroxide or the like. The preparations having the liquid properties within the range as defined hereinabove can be stored in an extremely stable manner for a long period of time without causing any coloring or depositing or precipitating.

In order to prepare the compositions for curing the affected abnormal tissues from the components as described hereinabove, it is necessary to formulate each of the components in accordance with the order and conditions of formulation, as will be described hereinafter, with the properties of each component taken into consideration.

Specifically, the compositions can be prepared by formulating sodium hydrogen sulfite, the water-soluble aluminum compound and citric acid or a salt thereof in an optional order into a formulation and adding tannic acid to the resulting formulation.

In addition, the polyvalent alcohol and/or the saccharide may be added to the formulation or a mixture prepared by adding tannic acid to the formulation.

Alternatively, the compositions for curing the affected abnormal tissues may be prepared by separately forming a formulation by admixing the water-soluble aluminum compound with citric acid or a salt thereof and a formulation by admixing tannic acid with the sodium hydrogen sulfite and then combining the two formulations together into the composition.

In this case, too, the polyvalent alcohol and/or the.saccharide may be added to either one or both of the formulations prepared in the alternative manner as has been. described hereinabove or the mixture obtained by combining the two formulations.

In summary, the methods for the preparation of the compositions for curing the affected abnormal tissues according to the present invention may comprise, as a method, adding tannic acid to the formulation prepared by admixing sodium hydrogen sulfite with all the components other than tannic acid and sodium hydrogen sulfite in an optional way or, as an alternative method, separately preparing the formulation by admixing the water-soluble aluminum compound with citric acid or a salt thereof and another formulation by admixing the tannic acid with the sodium hydrogen sulfite and then combining the formulation together with the other formulation into the mixture and, as needed, adding the polyvalent alcohol and/or saccharide to the formulation or formulations or to the mixture at any stage of preparation. By formulating each of the components in the manner as described hereinabove, the oxidation of tannic acid can be prevented as well as the direct reaction of tannic acid with the metal ions such as the aluminum ions derived from the water-soluble aluminum compound can also be prevented, thereby enabling the formation of the stable compositions for curing the affected abnormal tissues according to the present invention in a constant manner.

In the method for the preparation of the compositions for curing the affected abnormal tissues according to the present invention, the pH range of the compositions can be made, as needed, at any, stage of formulation or admixture of the components so as for the resulting injectable preparations to demonstrate the liquidpropertieswithin theabove-defined level, i.e. from pH1.0 to pH3.5. More specifically, when the compositions are prepared by adding tannic acid to the formulation prepared by admixing all the components other than tannic acid, the liquid properties of the injectable preparations can be adjusted at any or every stage of formulation or admixture by admixing the water-soluble aluminum compound and citric acid or a salt thereof with sodium hydrogen sulfite and admixing tannic acid to the resulting formulation and adding the polyvalent alcohol and/or saccharide to the resulting mixture as needed. Alternatively, when the compositions are prepared by admixing the formulation consisting of the water-soluble aluminum compound and citric acid or a salt thereof with the formulation consisting of the tannic acid and the sodium hydrogen sulfite, the liquid properties of the injectable preparations can also be adjusted at any or every stage of formulation or admixture to the above-defined level by admixing the resulting formulations together with each other to yield the mixture and adding the polyvalent alcohol and/or saccharide to the resulting mixture as needed.

In the methods for the preparation of the compositions for curing the affected abnormal tissues according to the present invention, it is preferred that any or every component of the composition is formulated with each other under atmosphere of an inert gas. As the inert gases to be used for this purpose, there may preferably be mentioned, for example, nitrogen gas.

Further, it is preferred that the compositions are prepared by formulating each of the components under conditions under which oxygen does not exist in an atmosphere or in water to be used in no substantial amount or in an amount restricted within the level that does not adversely affect the resulting compositions. Such level may be, for example, 1/1,000 or less with respect to the level in a stationary state.

The compositions thus prepared in the manner as described hereinabove are then filtered and sterilized by means of conventional methods and filled in a container such as colorless hard glass ampoules or glass bottles. They are then sterilized by high-pressure steam, as needed, and stored in a cold place.

In accordance with the method for the preparation of the compositions for curing the affected abnormal tissues, it is preferred that each or every step of the method be carried out in the presence of inert gases such as nitrogen gas. The oxygen dissolved in the injectable preparations is usually removed by conventional methods and the air present in the injectable preparations is replenished with nitrogen gas.

The compositions according to the present invention can be lyophilized by conventional methods. They also can be applied by dissolving the components in distilled water in situ. In this case, a solution prepared by dissolving the polyvalent alcohol and/or saccharide may also be used as a solution for use in dissolving the compositions in site.

The compositions for curing the affected abnormal tissues prepared according to the present invention have the peculiar properties of killing the affected abnormal tissues of the digestive organs, particularly the large intestine and rectum, upon contact with the locations of the tissues affected with the piles or hemorrhoids, such as internal piles or hemorrhoids, archoptoma or the like, the uplift affected tissues, the tissues affected with hepatic tumor and the like.

The compositions prepared according to the present invention can demonstrate the strong action of killing the cells of the affected abnormal tissues while holding the states or shapes of the cells. In other words, it is anticipated that the compositions can kill the cells of the affected abnormal tissues upon contact by application and then cause the killed cells to fall off from the normal tissues. By allowing the killed cells to fall off from the normal tissues, the compositions according to the present invention can sclerose the affected abnormal tissues and cure them in mild conditions.

### BEST MODE FOR CARRYING OUT THE INVENTION

The compositions for curing the affected abnormal tissues according to the present invention will be described in more detail by way of examples.

### Example No. 1:

The composition for curing the affected abnormal tissues according to this example contained the following components:

| | |
|---|---|
| Aluminum potassium sulfate | 400 mg |
| Sodium citrate | 150 mg |
| Sodium hydrogen sulfite | 15 mg |
| Tannic acid | 15 mg |
| Dextran 40 | 70 mg |
| Glycerin | 1,000 mg |
| Injectable distilled water | To make 10 ml |

The injectable distilled water prepared so as to comply with Pharmacopedia Japonica was treated to remove dissolved oxygen by heating it at 100 °C for five minutes. After the completion of heating, nitrogen gas was introduced into the treated water and allowed to cool to room temperature.

Separately, the total amount of dextran 40 was fully dissolved in an appropriate solvent in advance because it is less soluble to the solution for use.

The total amount of sodium hydrogen sulfite was dissolved into an appropriate amount of the injectable distilled water, and the resulting solution was added with the dextran 40 solution and then with sodium citrate, followed by the addition thereto of the total amounts of aluminum potassium sulfate and glycerin. To the resulting solution was added the total amount of tannic acid. The formulation of each of the components was carried out while nitrogen gas had been kept introduced thereinto. The resulting solution was stirred for about 30 minutes and filtered through a filter. Into the filtrate was introduced nitrogen gas to yield the objective composition. The composition had a pH of 2.7.

The resulting solution was then filled in glass vials or ampoules. Before and after filling, the air in the solution was replenished with nitrogen gas and the vials or ampoules were sealed, followed by storage in a cold dark place.

### Example No. 2:

The composition having the same components as the one prepared in Example No. 1 was prepared in substantially the same manner as in Example No. 1 with the exception that the dextran 40 solution, sodium citrate, aluminum potassium sulfate and glycerin were added in the order of sodium citrate, aluminum potassium sulfate, glycerin and the dextran 40 solution. The resulting composition had a pH of 2.6.

### Example No. 3:

The composition having the same components as the one prepared in Example No. 1 was prepared in substantially the same manner as in Example No. 1 with the exception that the dextran 40 solution, sodium citrate, aluminum potassium sulfate and glycerin were added in the order of aluminum potassium sulfate, glycerin, the dextran 40 solution and sodium citrate. The resulting composition had a pH of 2.7.

### Example No. 4:

The composition having the same components as the one prepared in Example No. 1 was prepared in substantially the same manner as in Example No. 1 with the exception that the dextran 40 solution, sodium citrate, aluminum potassium sulfate and glycerin were added in the order of glycerin, the dextran 40 solution, sodium citrate and aluminum potassium sulfate. The resulting composition had a pH of 2.7.

### Example No. 5:

The composition for curing the affected abnormal tissues according to this example contained the following components:

| | |
|---|---|
| Aluminum chloride | 400 mg |
| Tannic acid | 15 mg |
| Sodium citrate | 150 mg |
| Dextran 40 | 70 mg |
| Sodium hydrogen sulfite | 15 mg |
| Mannitol | 1,500 mg |
| Injectable distilled water | To make 10 ml |

The injectable distilled water was treated to remove dissolved oxygen by heating at 100 °C for five minutes. After the completion of heating, nitrogen gas was introduced into the treated water and allowed to cool to room temperature.

The total amount of sodium hydrogen sulfite was dissolved into an appropriate amount of the injectable distilled water thermally treated, and the resulting solution was added with the dextran 40 solution prepared separately and then with sodium citrate, followed the addition thereto of the total amounts of aluminum chloride and mannitol. To the resulting solution was then added the total amount of tannic acid yielding a solution. The formulation of each of the components was carried out while nitrogen gas had been kept introduced thereinto. The resulting solution was stirred for about 30 minutes, added with hydrochloric acid to adjust its pH to pH3.0 and filtered through a filter. Into the filtrate was introduced nitrogen gas to yield the objective composition.

The resulting solution was then filled in glass vials or ampoules. Before and after filling, the air in the solution was replenished with nitrogen gas and the vials or ampoules were sealed, followed by storage in a cold dark place.

### Example No. 6:

The composition of this example contained the components as follows:

| | |
|---|---|
| Aluminum sulfate | 400 mg |
| Tannic acid | 15 mg |
| Sodium citrate | 150 mg |
| Dextran 40 | 70 mg |
| Sodium hydrogen sulfite | 15 mg |
| Fructose | 2,000 mg |
| Injectable distilled water | To make 10 ml |

The injectable preparations were prepared by formulating each of the components in substantially the manner as in Example No. 5 with the exception that aluminum chloride was replaced with aluminum sulfate and mannitol was replaced with fructose. The resulting composition had a pH of 2.7.

### Example No. 7:

The composition of this example contained the components as follows:

| | |
|---|---|
| Aluminum carbonate | 200 mg |
| Tannic acid | 15 mg |
| Sodium citrate | 150 mg |
| Dextran 40 | 70 mg |
| Sodium hydrogen sulfite | 15 mg |
| Xylitol | 2,000 mg |
| Injectable distilled water | To make 10 ml |

The injectable preparations were prepared in substantially the same manner as in Example No. 5 with the exception that aluminum chloride was replaced with aluminum carbonate and mannitol was replaced with xylitol. The resulting composition was treated with sulfuric acid to give a pH of 2.7.

### Example No. 8:

The composition of this example contained the components as follows:

| | |
|---|---|
| Aluminum acetate | 200 mg |
| Tannic acid | 15 mg |
| Sodium citrate | 150 mg |
| Dextran 40 | 70 mg |
| Sodium hydrogen sulfite | 15 mg |
| Glucose | 3,000 mg |
| Injectable distilled water | To make 10 ml |

The injectable preparations were prepared in substantially the same manner as in Example No. 5 with the exception that aluminum chloride was replaced with aluminum'acetate and mannitol was replaced with glucose. The resulting composition was treated with sulfuric acid to give a pH of 2.7.

### Example No. 9:

The composition for curing the affected abnormal tissues according to this example contained the components as follows:

| | |
|---|---|
| Aluminum potassium sulfate | 400 mg |
| Tannic acid | 15 mg |
| Sodium citrate | 150 mg |
| Dextran 40 | 70 mg |
| Sodium hydrogen sulfite | 10 mg |
| Glycerin | 1,000 mg |
| Injectable distilled water | To make 10 ml |

The injectable preparations were prepared by formulating the above components in substantially the same manner as in Example No. 1. The resulting composition was adjusted with sulfuric acid to a pH of 2.7.

### Example No. 10:

The composition for curing the affected abnormal tissues according to this example contained the components as follows:

| | |
|---|---|
| Aluminum potassium sulfate | 400 mg |
| Tannic acid | 30 mg |
| Sodium citrate | 150 mg |
| Dextran 40 | 70 mg |
| Sodium hydrogen sulfite | 30 mg |
| Glycerin | 1,000 mg |
| Injectable distilled water | To make 10 ml |

The injectable preparations were prepared by formulating the above components in substantially the same manner as in Example No. 1. The resulting composition was adjusted with sulfuric acid to a pH of 2.8.

### Example No. 11:

The composition for curing the affected abnormal tissues according to this example contained the components as follows:

| | |
|---|---|
| Aluminum potassium sulfate | 400 mg |
| Tannic acid | 50 mg |
| Sodium citrate | 150 mg |
| Dextran 40 | 70 mg |
| Sodium hydrogen sulfite | 70 mg |
| Glycerin | 1,000 mg |
| Injectable distilled water | To make 10 ml |

The injectable preparations were prepared by formulating the above components in substantially the same manner as in Example No. 1. The resulting composition was adjusted with sulfuric acid to a pH of 2.7.

### Example No. 12:

The composition for curing the affected abnormal tissues according to this example contained the components as follows:

| | |
|---|---|
| Aluminum potassium sulfate | 400 mg |
| Tannic acid | 70 mg |
| Sodium citrate | 150 mg |
| Dextran 40 | 70 mg |
| Sodium hydrogen sulfite | 70 mg |
| Glycerin | 1,000 mg |
| Injectable distilled water | To make 10 ml |

The injectable preparations were prepared by formulating the above components in substantially the same manner as in Example No. 1. The resulting composition had a pH of 3.2.

### Example No. 13:

The composition for curing the affected abnormal tissues according to this example contained the components as follows:

| | |
|---|---|
| Aluminum potassium sulfate | 400 mg |
| Tannic acid | 90 mg |
| Sodium citrate | 150 mg |
| Dextran 40 | 70 mg |
| Sodium hydrogen sulfite | 100 mg |
| Glycerin | 1,000 mg |
| Injectable distilled water | To make 10 ml |

The injectable preparations were prepared by formulating the above components in substantially the same manner as in Example No. 1. The resulting composition was adjusted with sulfuric acid to a pH of 2.7.

### Example No. 14:

The composition for curing the affected abnormal tissues according to this example contained the components as follows:

| | |
|---|---|
| Aluminum potassium sulfate | 400 mg |
| Tannic acid | 100 mg |
| Sodium citrate | 150 mg |
| Dextran 40 | 70 mg |
| Sodium hydrogen sulfite | 100 mg |
| Glycerin | 1,000 mg |
| Injectable distilled water | To make 10 ml |

The injectable preparations were prepared by formulating the above components in substantially the same manner as in Example No. 1. The resulting composition was adjusted with sulfuric acid to a pH of 2.7.

### Example No. 15:

The composition for curing the affected abnormal tissues according to this example contained the components as follows:

| | |
|---|---|
| Aluminum potassium sulfate | 400 mg |
| Tannic acid | 120 mg |
| Sodium citrate | 150 mg |
| Sodium hydrogen sulfite | 140 mg |
| Glycerin | 1,200 mg |
| Injectable distilled water | To make 10 ml |

The injectable preparations were prepared by formulating the above components in substantially the same manner as in Example No. 1. The resulting composition was adjusted with sulfuric acid to a pH of 2.7.

### Example No. 16:

The composition for curing the affected abnormal tissues according to this example contained the components as follows:

| | |
|---|---|
| Aluminum potassium sulfate | 400 mg |
| Tannic acid | 15 mg |
| Sodium citrate | 300 mg |
| Sodium hydrogen sulfite | 20 mg |
| Mannitol | 3,000 mg |
| Injectable distilled water | To make 10 ml |

The injectable preparations were prepared in substantially the same manner as in Example No. 1 with the exception that dextran was not used and glycerin was replaced with mannitol. The resulting composition was adjusted with sulfuric acid to a pH of 2.7.

### Example No. 17:

The composition for curing the affected abnormal tissues according to this example contained the components as follows:

| | |
|---|---|
| Aluminum potassium sulfate | 400 mg |
| Sodium citrate | 50 mg |
| Sodium hydrogen sulfite | 15 mg |
| Tannic acid | 15 mg |
| Glycerin | 1,100 mg |
| Injectable distilled water | To make 10 ml |

The injectable distilled water prepared so as to comply with Pharmacopedia Japonica was treated to remove dissolved oxygen by heating at 100 °C for five minutes. After the completion of heating, nitrogen gases were. introduced into the treated water and allowed to cool to room temperature.

The total amount of sodium hydrogen sulfite was dissolved into an appropriate amount of the injectable distilled water, and the resulting solution was added with sodium citrate, followed by the addition thereto of the total amounts of aluminum potassium sulfate and glycerin. To the resulting solution was added the total amount of tannic acid. The formulation of each of the components was carried out while nitrogen gas had been kept introduced -thereinto. The resulting solution was stirred for about 30 minutes, adjusted with sulfuric acid to demonstrate a pH of 3.1, and filtered through a filter. Into the filtrate was introduced nitrogen gas to yield the objective composition.

The resulting solution was then filled in glass vials or ampoules. Before and after filling, the air in the solution was replenished with nitrogen gas and the vials or ampoules were sealed, followed by storage in a cold dark place.

### Example No. 18:

The composition for curing the affected abnormal tissues according to this example contained the components as follows:

| | |
|---|---|
| Aluminum potassium sulfate | 400 mg |
| Sodium citrate | 100 mg |
| Sodium hydrogen sulfite | 15 mg |
| Tannic acid | 15 mg |
| Dextran 40 | 100 mg |
| Injectable distilled water | To make 10 ml |

The injectable distilled water prepared so as to comply with Pharmacopedia Japonica was treated to remove dissolved oxygen by heating at 100 °C for five minutes. After the completion of heating, nitrogen gas as introduced into the treated water and the solution allowed to cool to room temperature.

The total amount of sodium hydrogen sulfite was dissolved into an appropriate amount of the injectable distilled water, and the resulting solution was added with the total amounts of the dextran 40 solution separately prepared and sodium citrate, followed by the addition thereto of the total amount of aluminum potassium sulfate. To the resulting solution was added the total amount of tannic acid. The formulation of each of the components was carried out while nitrogen gas had been kept introduced thereinto. The resulting solution was stirred for about 30 minutes, adjusted with sulfuric acid to give a pH of 2.8, and filtered through a filter. Into the filtrate was introduced nitrogen gas to yield the objective composition.

The resulting solution was then filled in glass vials or ampoules. Before and after filling, the air in the solution was replenished with nitrogen gas and the vials or ampoules were sealed, followed by storage in a cold dark place.

### Example No. 19:

This example provided the injectable preparations for curing the affected abnormal tissues for use in site, which contained the components as follows:

| Agent I: | |
|---|---|
| Tannic acid | 15 mg |
| Sodium hydrogen sulfite | 15 mg |
| Injectable distilled water | To make 10 ml |

| Agent II: | |
|---|---|
| Aluminum potassium sulfate | 400 mg |
| Sodium citrate | 150 mg |
| Dextran 40 | 70 mg |
| Glycerin | 1,000 mg |
| Injectable distilled water | To make 10 ml |

The above Agent I was prepared by adding the total amount of tannic acid in the presence of nitrogen gas to a solution prepared by dissolving sodium hydrogen sulfite in the injectable distilled water. On the other hand, the above agent II was prepared by dissolving sodium citrate in the injectable distilled water in the presence of nitrogen gas and adding thereto aluminum potassium sulfate in the presence of nitrogen gas, followed by addition thereto of dextran and glycerin. The Agents I and II prepared in the above manner were combined into injectable preparations for use in situ by adding the Agent II to the Agent I. The injectable preparations were adjusted with sulfuric acid to a pH of 2.7.

### Example No. 20:

This example provided the injectable preparations for curing the affected abnormal tissues for use in site which contained the components as follows:

| Agent I: | |
|---|---|
| Aluminum potassium sulfate | 400 mg |
| Sodium citrate | 150 mg |
| Sodium hydrogen sulfite | 20 mg |
| Injectable distilled water | To make 10 ml |

| Agent II: | |
|---|---|
| Tannic acid | 20 mg |
| Glycerin | 1,200 mg |
| Injectable distilled water | To make 10 ml |

The above Agent I was prepared by adding the total amounts of sodium hydrogen sulfite and sodium citrate in the presence of nitrogen gas to the injectable distilled water and then aluminum potassium sulfate to the resulting solution in the presence of nitrogen gas. Separately, the above agent II was prepared by dissolving glycerin and tannic acid in this order in the injectable distilled water in the presence of nitrogen gas. The Agents I and II prepared in the manner as described hereinabove were combined in situ for use as injectable preparations by adding the Agent II to the Agent I. The injectable preparations were adjusted with sulfuric acid, as needed, to a pH of 2.7.

### Example No. 21:

This example provided the injectable preparations for curing the affected abnormal tissues for use in site which contained the components as follows:

| Agent I: | |
|---|---|
| Aluminum potassium sulfate | 400 mg |
| Sodium citrate | 150 mg |
| Injectable distilled water | To make 10 ml |

| Agent II: | |
|---|---|
| Tannic acid | 50 mg |
| Sodium hydrogen sulfite | 100 mg |
| Injectable distilled water | To make 10 ml |

The above Agent I was prepared by dissolving sodium citrate in injectable distilled water and then aluminium potassium sulfate in the resulting solution in the presence of nitrogen gas. The above Agent II was prepared by dissolving sodium hydrogen sulfite and tannic acid in injectable distilled water in the presence of nitrogen gas. The resulting Agent I was lyophilised in a conventional manner. An injection solution was prepared by adding the Agent II to the agent I upon use in-situ and used for stability tests as will be described later. The injectable preparation was adjusted to a pH of 2.7.

### Example No. 22:

This example provided the injectable preparations for curing the affected abnormal tissues for use in situ which contained the components as follows:

| Agent I: | |
|---|---|
| Aluminum potassium sulfate | 400 mg |
| Sodium citrate | 150 mg |
| Tannic acid | 15 mg |
| Sodium hydrogen sulfite | 15 mg |
| Injectable distilled water | To make 10 ml |

| Agent II: | |
|---|---|
| Glycerin | 1,000 mg |
| Dextran | 70 mg |
| Injectable distilled water | To make 10 ml |

The above Agent I was prepared by adding the total amount of sodium citrate to the injectable distilled water and then aluminum potassium sulfate to the resulting solution in the presence of nitrogen gas. To the resulting solution was added a solution prepared by dissolving the total amounts of sodium hydrogen sulfite and tannic acid in the injectable distilled water in the presence of nitrogen gas. The resulting Agent I had a pH of 2.7 and was lyophilized in conventional manner, followed by storage for one month in a cold dark place.

The injectable preparations were prepared for use in situ by adding the Agents II to the Agent I and used for tests for stability of the preparations in a way as will be described hereinafter.

### Comparative Example No. 1:

The composition prepared in Example No. 1 was adjusted with sodium hydroxide to pH4.7 for comparative purposes.

### Comparative Example No. 2:

The composition was prepared by adding dextran, aluminum potassium sulfate, glycerin and tannic acid in this consecutive order in the injectable distilled water in the presence of nitrogen gas. The resulting injectable preparations were adjusted with sulfuric acid to pH2.7.

### Comparative Example No. 3:

The injectable preparation of this comparative example was prepared which contained the following components:

| | |
|---|---|
| Aluminum potassium sulfate | 4.0 g |
| Tannic acid | 0.15 g |
| Sodium citrate | 1.5 g |
| Dextran | 10 ml |
| Trichlorobutanol | 0.5 g |
| Glycerin | 10 ml |
| Injectable distilled water | To make 100 ml |

The injectable preparation was prepared by adding each of the above components to the injectable distilled water in the manner as enumerated hereinabove and the resulting preparation was adjusted with sodium hydroxide to pH4.7.

### Comparative Example No. 4:

The sclerosing agent having the same composition as disclosed in Japanese Patent Unexamined Publication (Kokai) No. 4-225,920 as the Comparative Preparation Formulation Example No. 1 was prepared in substantially the same manner as disclosed in this publication. More specifically, a composition having the same components was formulated in the same order and in substantially the same manner as disclosed in this publication to give an injectable preparation which was then adjusted with sodium hydroxide to pH4-5. The resulting injectable preparation was filled by a 10 ml portion in each of colorless hard glass ampoules which in turn were treated in order to replenish the air in the ampoules with nitrogen gas and then sterilized with high pressure steam in conventional manner.

### Comparative Example No. 5:

The sclerosing agent having the same composition as disclosed in Japanese Patent Unexamined Publication (Kokai) No. 4-225,920 as the Comparative Preparation Formulation Example No. 2 was prepared in substantially the same manner as disclosed in this publication. More specifically, the composition having the same components was formulated in substantially the same manner as disclosed in this publication to yield injectable preparations which were then adjusted with sodium hydroxide to pH4-5. The resulting injectable preparations were filled by a 10 ml portion in each of colorless hard glass ampoules which in turn were treated in order to replenish the air in the ampoules with nitrogen gas and then sterilized with high pressure steam in conventional manner.

### Comparative Example No. 6:

The sclerosing agent having the same composition as disclosed in Japanese Patent Unexamined Publication (Kokai) No. 4-225,920 as the Comparative Preparation Formulation Example No. 5 was prepared in substantially the same manner as disclosed in the publication. More specifically, the composition having the same components was formulated in the same order and in substantially the same manner as disclosed in the publication to give the composition which was then adjusted with sulfuric acid to pH2.7. The resulting aqueous solution was filled by a 50 ml portion in each of colorless hard glass vials which in turn were treated in order to replenish the gases in the vials with nitrogen gas and then sterilized with high pressure steam in conventional manner.

### Comparative Example No. 7:

Into a test tube were poured simultaneously the same components in the same amounts as Example No. 1, and injectable distilled water was added with stirring to the mixture to make the total amount 10 ml. The resulting solution became turbid in white color and caused precipitating as the time passed by.

### Comparative Example No. 8:

For the preparation of the composition having the same components and containing the same amounts, respectively, as Example No. 1 above, dextran and glycerin were admixed with injectable distilled water, followed by the addition of aluminum potassium sulfate and tannic acid to the resulting mixture. To the resulting solution were added sodium citrate and sodium hydrogen sulfite to give an injectable preparation. It was found that this solution turned turbid in white color at the time when tannic acid was added and it soon caused precipitates in a dark brown color.

### Comparative Example No. 9:

A solution (Agent I) was prepared by dissolving 400 mg of aluminum potassium sulfate and 400 mg of tannic acid in 10 ml of injectable distilled water in the presence of nitrogen gas. Separately, a solution (Agent II) was prepared by dissolving 70 mg of dextran, 1,000 mg of glycerin, 15 mg of sodium citrate and 15 mg of sodium hydrogen sulfite in this order in 10 ml of injectable distilled water in the presence of nitrogen gases. The injectable preparations were prepared by admixing Agent I with Agent II. The injectable preparation was found to turn turbid in pale brown color at the time when tannic acid was added to aluminum potassium sulfate in the Agent I and the resulting solution caused precipitates in dark brown color within short.

The injectable preparations prepared in the manner as described hereinabove were subjected to tests for stability of preparations.

### Tests for Stability of Preparations:

After the injectable preparations prepared in the above-mentioned Examples were adjusted to give the pH ranges as defined hereinabove, five colorless hard glass vials were aseptically filled with 10 ml of each of the compositions and the air in the vials was replenished with nitrogen gas under vacuum conditions. The vials were then exposed to white light with 1,000 lux at 40° C and visual observations were made as to coloring and the extent of depositing. Before carrying out the tests for stability of injectable preparations, each of the compositions was found colorless or transparent, yellowish and slightly viscous. The test results are shown in Table 1 below.

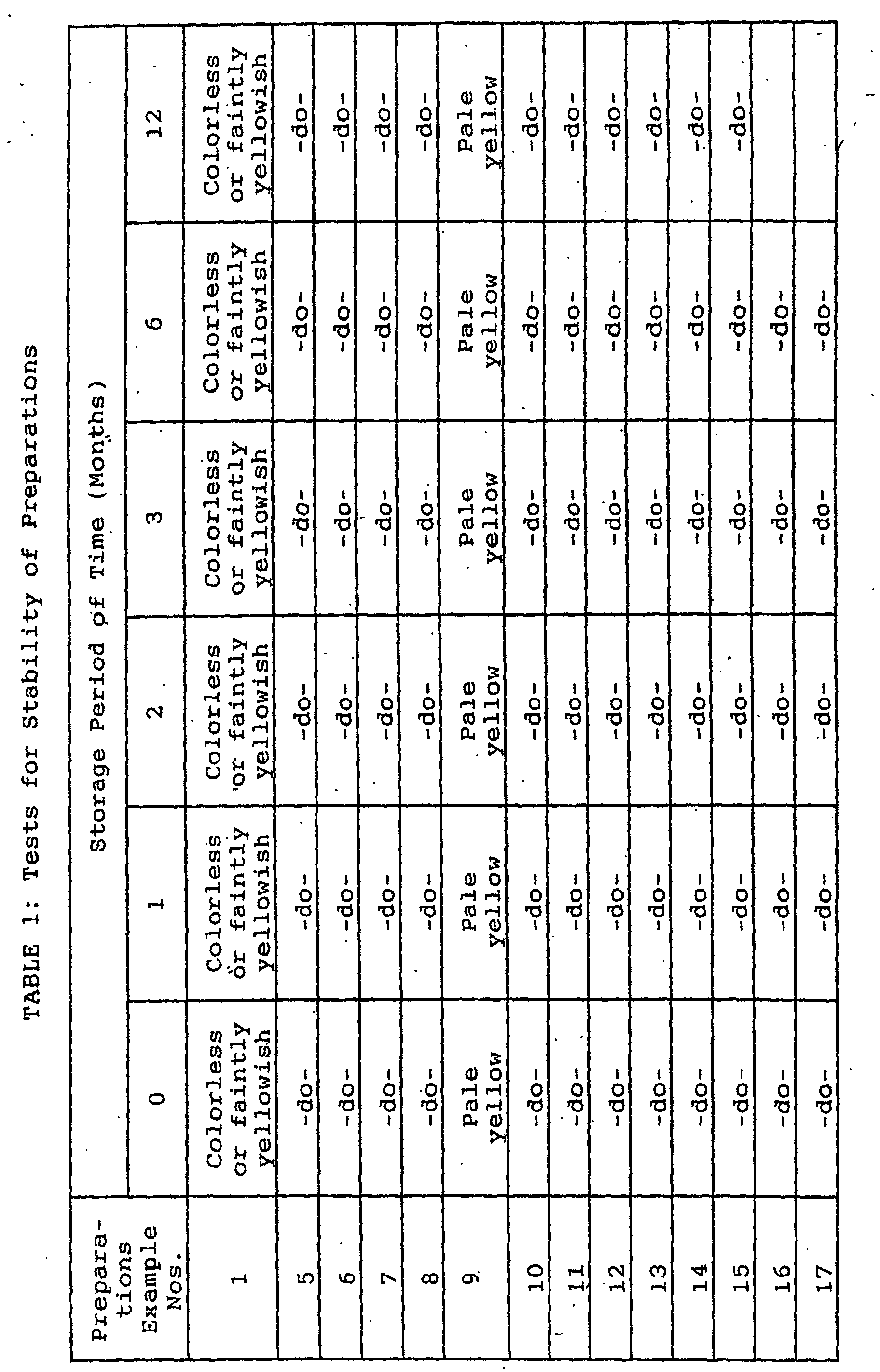

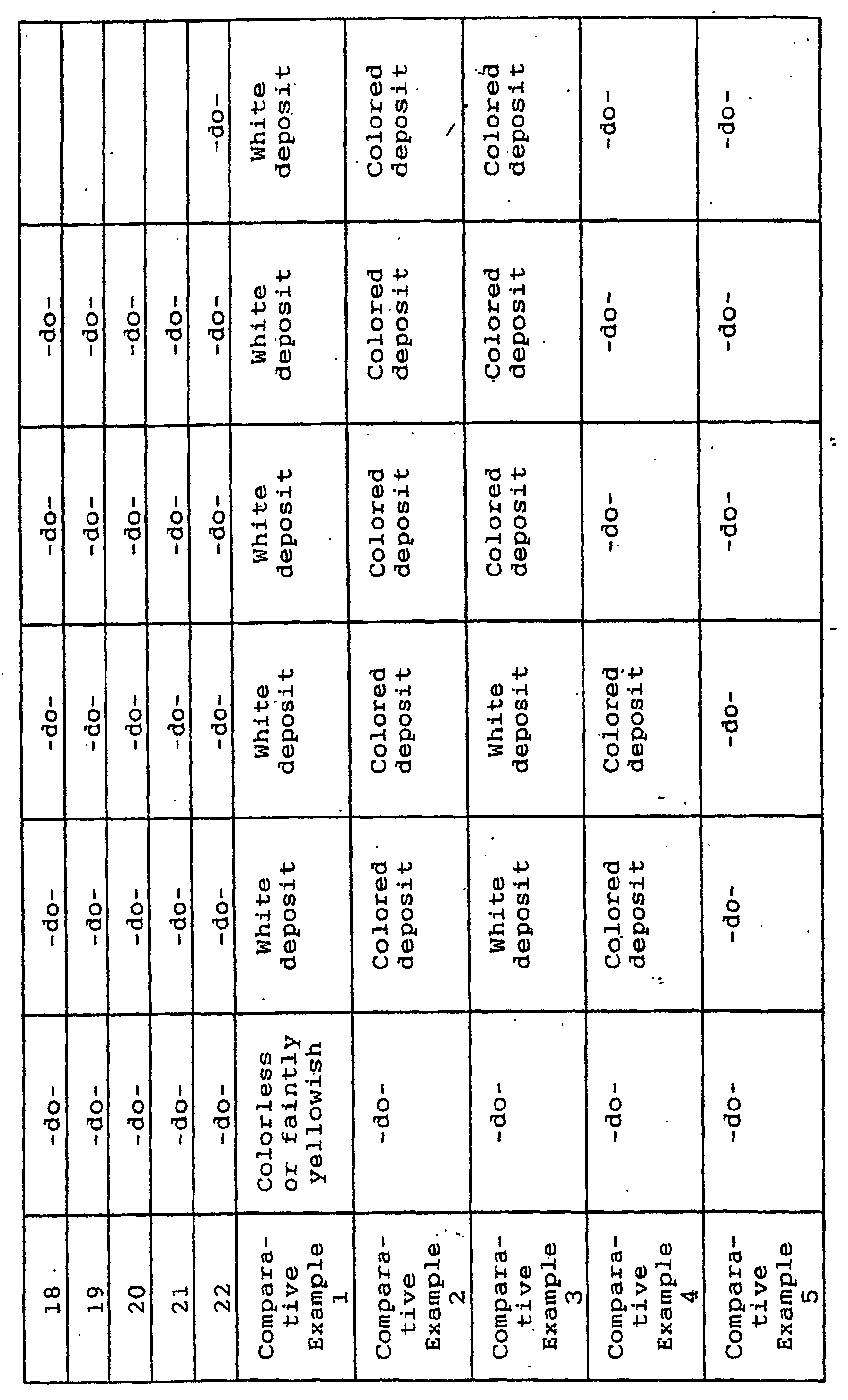

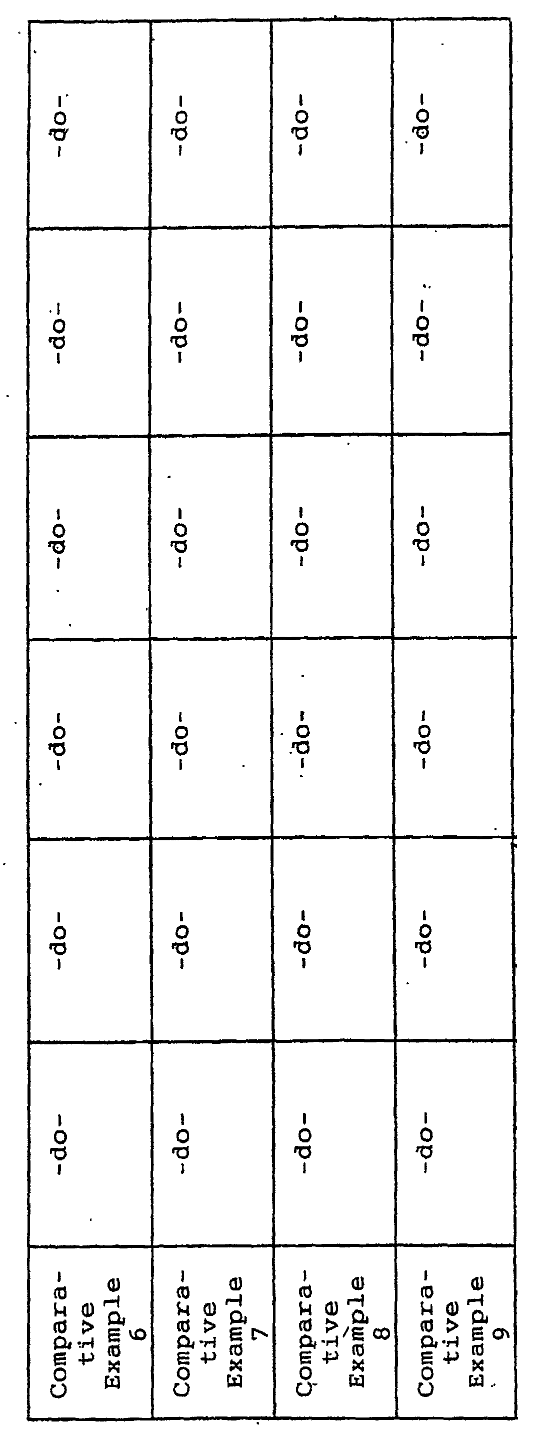

Table 2 below shows the results of the tests for stability of the injectable preparations in substantially the same manner as in Table 1 above with the exception that the temperature for storage was set to 60°C.

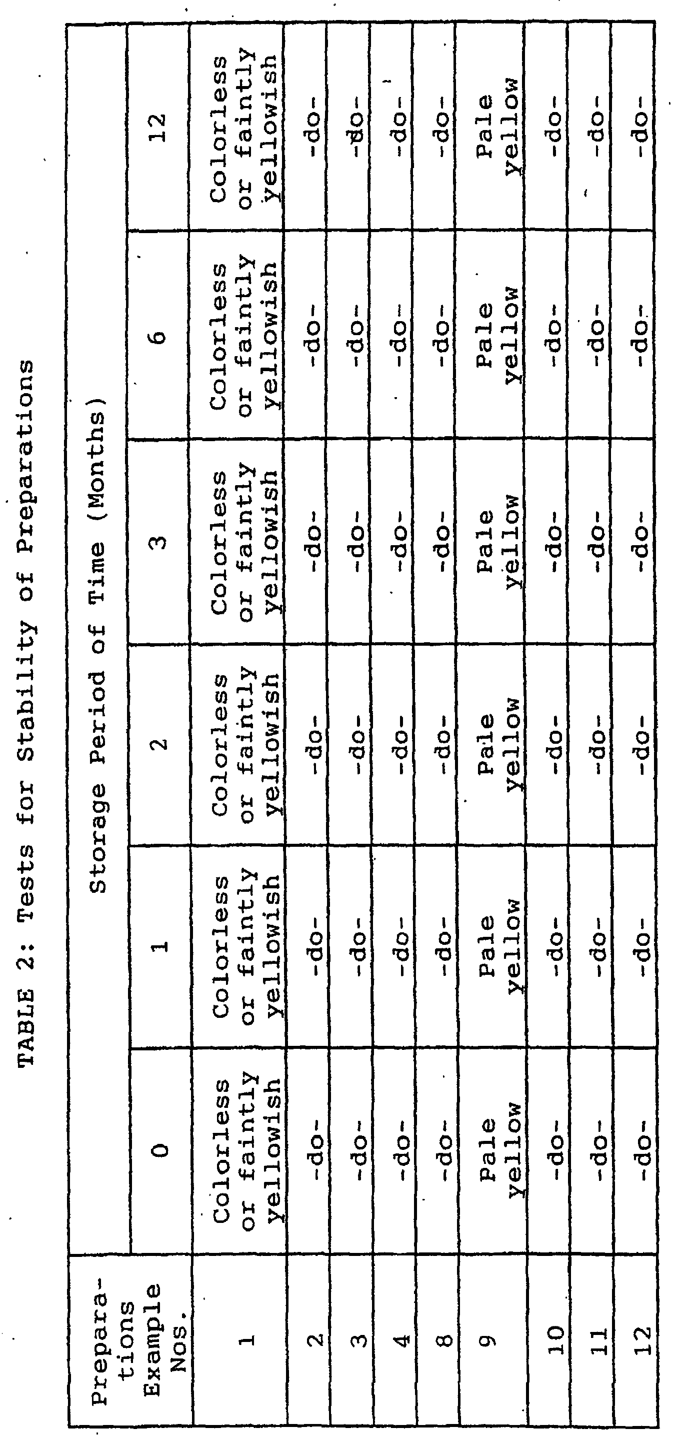

A description.will made of experiments for the cytotoxic action of the compositions for curing the affected abnormal tissues according to the present invention.

For the injectable preparations obtained in Example No. 1, a comparative review of the cytotoxic effects was made using the endothelial cells of the venous vessel of the human funiculus (primary culture), the fibroblast of the human normal diploid (primary culture) and the rat muscle blast cells (cell culture). As comparative chemicals, there were employed Paoscle™ (containing phenol as a major active ingredient) as an agent for sclerosing the internal piles and Oldamin™ (containing monoethanolamine oleate as a major active ingredient), Aethoxysklerol™ (containing polidocanol as a major active ingredient) and ethanol (containing ethanol as a major active ingredient) as agents for sclerosing esophageal varix.

The method for determining the death of the cells was based on the trypan blue dyeing method.

### Experiment No. 1:

The method for experiment was as follows. Each kind of the cells was inoculated in a culture solution prepared by adding 10% fetal bovine serum to a mixture of DMEM with HAMF12 (1:1) and cultured at 37°C in the presence of 5% CO₂.

Then, the resulting culture was digested with trypsin to give a cells floating solution which in turn was mixed with an equal amount of the test agent. The resulting mixture was allowed to stand at 37°C for 10 minutes and mixed with an equal amount of 0.3% trypan blue solution. 15 minutes after the addition of the trypan blue solution, the number of the living cells (not dyed) and the dead cells (dyed blue) was counted.

As a control, a 0.9% sodium chloride solution was used.

The test results are shown in Table 3 below.

**TABLE 3:**

| RATES OF LIVING CELLS | | | |
|---|---|---|---|
| TEST AGENTS | RATES OF LIVING CELLS (%) | | |
| | CELL 1 | CELL 2 | CELL 3 |
| COMPOSITION OF THE PRESENT INVENTION | 96.8 | 90.3 | 95.0 |
| PAOSCLE™ | 79.8 | 89.3 | 87.0 |
| OLDAMIN™ | 0.0 | 0.0 | 0.0 |
| AETHOXYSKLEROL™ | 0.0 | 0.0 | 0.0 |
| ETHANOL | 0.0 | 0.0 | 0.0 |
| CONTROL (0.9% SODIUM CHLORIDE SOLUTION) | 97.0 | 91.7 | 94.0 |
| NOTES: CELL 1: Endothelial cells of the venous vessel of the human funiculus (primary culture) | | | |
| CELL 2: Fibroblast of the human normal diploid (primary culture) | | | |
| CELL 3: Rat muscle blast cells (cell culture) | | | |

As is apparent from the test results as shown in Table 3 above, the 0.9% sodium chloride solution used as the control and Paoscle™ did not cause killing a majority of the cells tested, while Oldamin™, Aethoxysklerol™ and ethanol used as the agent for sclerosing the esophageal varix caused death of the entire number (100%) of the cells.

Among the living cells used in the above tests, the fibroblast of the human normal diploid was cultured again. More specifically, the living cells were washed and inoculated in a culture medium solution containing the same culture medium as used before, followed by the culture at 37°C for 24 hours. As a result, it was found that no living cells were counted at all out of the cells exposed to the composition according to the present invention, on the one hand, and that 95% of the cells were found alive and kept growing out of the cells treated with the 0.9% sodium chloride solution and 80% of the cells were alive and kept growing out of the cells treated with Paoscle™, on the other hand.

### INDUSTRIAL UTILIZATION OF THE INVENTION

The compositions according to the present invention are constant and stable in formulation as well as effective for curing the affected abnormal tissues of the digestive system, particularly the large intestine and rectum, because the compositions comprising substantially the water-soluble aluminum compound, citric acid or a salt thereof tannic acid and sodium hydrogen sulfite are adjusted within a pH range of from pH1.0 to pH3.5 in such a manner that tannic acid does not react with the metal ions, such as aluminum ions, derived from the water-soluble aluminum compound and no formation of precipitates are caused.

Further, the compositions for curing the affected abnormal tissues according to the present invention can be made more stable in formulation by adding the polyvalent alcohol and/or saccharide, as needed, to the compositions substantially comprising the water-soluble aluminum compound, citric acid or a salt thereof, tannic acid and sodium hydrogen sulfite.

In addition, in preparing the compositions for curing the affected abnormal tissues according to the present invention, tannic acid is prevented from causing precipitating as a result of its reaction with the metal ions, such as aluminum ions, decomposed from the water-soluble aluminum compound, by adding it to a formulation consisting substantially of the water-soluble aluminum compound, citric acid or a salt thereof and sodium hydrogen sulfite, whereby the stable compositions haying the constant formulation can be prepared.

Furthermore, it is noted that the admixture of a first formulation comprising the water-soluble aluminum compound and citric acid or a salt thereof with a second formulation comprising tannic acid and sodium hydrogen sulfite can lead to the preparation of the compositions according to the present invention, which can offer the advantages in preparing the compositions for curing the affected abnormal tissues according to the present invention that the reaction of tannic acid with the metal ions such as aluminum ions decomposed from the water-soluble aluminum compound can be controlled more readily so as to cause no precipitating.

It is further advantageous that the optional addition of the polyvalent alcohol and/or saccharide to the compositions consisting substantially of the water-soluble aluminum compound, citric acid or a salt thereof, tannic acid and sodium hydrogen sulfite can contribute greatly to making the compositions more stable and preparing the compositions in a ready way because the timing of the addition can be chosen in an optional way.

Furthermore, in preparing the compositions according to the method according to the present invention, it is extremely advantageous to enable the pH range of the 'compositions for curing the affected abnormal tissues to be adjusted at an appropriate stage of formulation of each of the components, particularly in terms of making it easy to prevent tannic acid from being oxidized and making it to prevent tannic acid from reacting with the metal ions derived from the water-soluble aluminum compound.

The present invention can offer the further advantage that the compositions according to the present invention can be made stable in formulation by formulating each of the components at an appropriate stage under atmosphere of inert gases.

Likewise, it is beneficial in preparing the stable compositions for curing the affected abnormal tissues according to the present invention having the constant and/stable formulation that each of the components is formulated under the condition under which no substantial amount or an extremely limited amount of oxygen exists in an atmosphere or in a liquid state because such condition can contribute particularly to a prevention of tannic acid from being oxidized.

In addition, it is very beneficial in preparing the stable compositions for curing the affected abnormal tissues according to the present invention having the constant formulation that a solution containing the polyvalent alcohol and/or saccharide can be added to or mixed with the first formulation comprising the water-soluble aluminum compound and citric acid or a salt thereof and/or the second formulation comprising tannic acid and sodium hydrogen sulfite or a mixture of the first formulation with the second formulation immediately .before administration, because this way of preparing the injectable preparations can prevent each of the components from being changed or transformed according to a lapse of time.

The compositions prepared according to the present invention can offer the further advantage that the injectable preparations can be stored in a stable state for a long period of time because it is possible to adjust the pH range of the compositions after all the components have been formulated. In addition, it is beneficial for stabilizing the compositions for a long period of time that the compositions can be stored under atmosphere of inert gas.

## Claims

1. A method for the preparation of a composition for curing affected abnormal tissues comprising substantially a water-soluble aluminum compound, citric acid or a salt thereof, tannic acid and sodium hydrogen sulfite and having a pH of said composition set to range from pH 1.0 to pH 3.5, **characterized in that** tannic acid is mixed with a mixture prepared by formulating said water-soluble aluminum compound, said citric acid or its salt and said sodium hydrogen sulfite in an optional order.

2. A method for the preparation of a composition for curing affected abnormal tissues as claimed in claim 1, wherein a polyvalent alcohol and/or a saccharide is added to said mixture prepared by formulating said sodium hydrogen sulfite, said water-soluble aluminum compound and/or said citric acid or a salt thereof.

3. A method for the preparation of a composition for curing affected abnormal tissues **characterized in that** tannic acid is admixed with a formulation prepared by mixing a water-soluble aluminum compound with citric acid or a salt thereof and having a pH of said composition set to range from pH 1.0 to 3.5, wherein sodium hydrogen sulfite is admixed with tannic acid or said formulation to yield said composition.

4. The method as claimed in claim 3, wherein a mixture of tannic acid with sodium hydrogen sulfite is mixed with the formulation of the water-soluble aluminum compound containing citric acid or a salt thereof.

5. The method as claimed in claim 3, wherein tannic acid is mixed with the formulation of the water-soluble aluminium compound and citric acid or a salt thereof containing sodium hydrogen sulfite.

6. A method for the preparation of a composition for curing affected abnormal tissues as claimed in claim 3, wherein a polyvalent alcohol and/or a saccharide are or Is added to each of sold tannic acid and said formulation or either one of said tannic acid and said formulation or to a mixture of tannic acid with said formulation.

7. A method as claimed in any of claims 1 to 6, wherein said water-soluble aluminum compound is selected from aluminum chloride, aluminum sulfate, aluminum carbonate, aluminum acetate, aluminum nitrate, aluminum lactate, aluminum tartrate, aluminum sallcylate, aluminum sodium sulfate, aluminum potassium sulfate, aluminum cesium sulfate and aluminum ammonium sulfate.

8. A method as claimed in any of claims 1 to 7, wherein said water-soluble aluminum compound is incorporated at a rate of from 1 % to 10 % as an effective concentration in preparations.

9. A method as claimed in any one of claims 1 to 6, wherein said tannic acid is incorporated at a rate of from 0.01 % to 2.0 % as an effective concentration in preparations.

10. A method as claimed in any one of claims 1 to 6, wherein said citric acid or a salt thereof is sodium citrate.

11. A method as claimed in any on e of claims 1 to 6, wherein said citric acid or a salt thereof is incorporated at a rate of from 10 % to 80 % with respect to the amount of said water-soluble aluminum compound.

12. A method as claimed in any one of claims 1 to 6, wherein said sodium hydrogen sulfite is contained at a rate of from 50 % to 200 % with respect to the amount of said tannic acid.

13. A method as claimed in claim 12, wherein said sodium hydrogen suffite is incorporated at a rate of from 70 % to 150 % with respect to the amount of said tannic acid.

14. A method as claimed in claim 2 or 6, wherein said polyvalent alcohol and/or said saccharlde is selected from mannitol, fructose, xylitol, glucose, galactose, mannose, lactose and glycerin.

15. A method as claimed in claim 14, wherein said polyvalent alcohol and/or said saccharide are or is incorporated at a rate of from 3 % to 20 % as an effective concentration in preparations.

## Patentansprüche

1. Verfahren für die Herstellung einer Zusammensetzung zur Heilung von geschädigten, abnormalen Geweben, wobei die Zusammensetzung im wesentlichen eine wasserlösliche Aluminiumverbindung, Zitronensäure oder ein Salz davon, Tannin und Natriumhydrogensulfit umfasst sowie einen auf den Bereich von pH 1,0 bis pH 3,5 eingestellten pH hat, **dadurch gekennzeichnet, dass** das Tannin mit einer Mischung gemischt wird, die durch Formulierung der wasserlöslichen Aluminiumverbindung, der Zitronensäure oder eines Salzes davon und des Natriumhydrogensulfits in einer optionalen Reihenfolge hergestellt wird.

2. Verfahren für die Herstellung einer Zusammensetzung zur Heilung von geschädigten, abnormalen Geweben nach Anspruch 1, wobei ein polyvalenter Alkohol und/oder ein Saccharid der Mischung zugesetzt wird, die durch Formulierung des Natriumhydrogensulfits, der wasserlöslichen Aluminiumverbindung und/oder der Zitronensäure oder eines Salzes davon hergestellt wird.

3. Verfahren für die Herstellung einer Zusammensetzung zur Heilung von geschädigten, abnormalen Geweben, **dadurch gekennzeichnet, dass** Tannin einer Formulierung beigemischt wird, die durch Mischen einer wasserlöslichen Aluminiumverbindung mit Zitronensäure oder einem Salz davon hergestellt wird, und die Zusammensetzung einen auf den Bereich von pH 1,0 bis pH 3,5 eingestellten pH hat, wobei Natriumhydrogensulfit dem Tannin oder der Formulierung beigemischt wird, um die Zusammensetzung zu erzielen.

4. Verfahren nach Anspruch 3, wobei eine Mischung von Tannin mit Natriumhydrogensulfit mit der Formulierung der wasserlöslichen Aluminiumverbindung, die Zitronensäure oder ein Salz davon enthält, gemischt wird.

5. Verfahren nach Anspruch 3, wobei Tannin mit der Formulierung der wasserlöslichen Aluminiumverbindung und der Zitronensäure oder einem Salz davon gemischt wird, wobei die Formulierung das Natriumhydrogensulfit enthält.

6. Verfahren für die Herstellung einer Zusammensetzung zur Heilung von geschädigten, abnormalen Geweben nach Anspruch 3, wobei ein polyvalenter Alkohol und/oder ein Saccharid sowohl dem Tannin als auch der Formulierung, oder dem Tannin oder der Formulierung, oder einer Mischung von Tannin und der Formulierung zugesetzt wird oder werden.

7. Verfahren nach einem der Ansprüche 1 bis 6, wobei die wasserlösliche Aluminiumverbindung aus Aluminiumchlorid, Aluminiumsulfat, Aluminiumcarbonat, Aluminiumacetat, Aluminiumnitrat, Aluminiumlactat, Aluminiumtartrat, Aluminiumsalicylat, Aluminiumnatriumsulfat, Aluminiumkaliumsulfat, Aluminiumcaesiumsulfat und Aluminiumammoniumsulfat ausgewählt wird.

8. Verfahren nach einem der Ansprüche 1 bis 7, wobei die wasserlösliche Aluminiumverbindung in einer Menge von 1 % bis 10 % als effektive Konzentration in den Zubereitungen eingelagert wird.

9. Verfahren nach einem der Ansprüche 1 bis 6, wobei das Tannin in einer Menge von 0,01 % bis 2 % als effektive Konzentration in den Zubereitungen eingelagert wird.

10. Verfahren nach einem der Ansprüche 1 bis 6, wobei die Zitronensäure oder ein Salz davon Natriumcitrat ist.

11. Verfahren nach einem der Ansprüche 1 bis 6, wobei die Zitronensäure oder ein Salz davon in einer Menge von 10 % bis 80 %, bezogen auf die Menge der wasserlöslichen Aluminiumverbindung, eingelagert wird.

12. Verfahren nach einem der Ansprüche 1 bis 6, wobei das Natriumhydrogensulfit in einer Menge von 50 % bis 200 %, bezogen auf die Menge des Tannins, vorhanden ist.

13. Verfahren nach Anspruch 12, wobei das Natriumhydrogensulfit in einer Menge von 70 % bis 150 %, bezogen auf die Menge des Tannins, eingelagert wird.

14. Verfahren nach Anspruch 2 oder 6, wobei der polyvalente Alkohol und/oder das Saccharid aus Mannit, Fructose, Xylitol, Glucose, Galactose, Mannose, Lactose und Glycerin ausgewählt wird.

15. Verfahren nach Anspruch 14, wobei der polyvalente Alkohol und/oder das Saccharid in einer Menge von 3 % bis 20 % als effektive Konzentration in den Zubereitungen eingelagert wird oder werden.

## Revendications

1. Procédé pour la préparation d'une composition pour traiter les tissus anormaux atteints comprenant substantiellement un composé d'aluminium soluble dans l'eau, de l'acide citrique ou un de ses sels, de l'acide tannique et de l'hydrogénosulfite de sodium et ladite composition ayant un pH se situant dans un intervalle de pH 1,0 à pH 3,5, **caractérisé en ce que** l'acide tannique est mélangé avec un mélange préparé en formulant ledit composé d'aluminium soluble dans l'eau, ledit acide citrique ou son sel et ledit hydrogénosulfite de sodium dans un ordre facultatif.

2. Procédé pour la préparation d'une composition pour traiter les tissus anormaux atteints selon la revendication 1, dans lequel un polyalcool et/ou un glucide sont ou est ajouté(s) audit mélange préparé en formulant ledit hydrogénosulfite de sodium, ledit composé d'aluminium soluble dans l'eau et/ou ledit acide citrique ou un de ses sels.

3. Procédé pour la préparation d'une composition pour traiter des tissus anormaux atteints **caractérisé en ce que** l'acide tannique est mélangé avec une formulation préparée en mélangeant un composé d'aluminium soluble dans l'eau avec de l'acide citrique ou un de ses sels et ladite composition ayant un pH se situant dans un intervalle de pH 1,0 à pH 3,5, dans lequel l'hydrogénosulfite de sodium est mélangé avec de l'acide tannique ou avec ladite formulation pour produire ladite composition.

4. Procédé selon la revendication 3, dans lequel un mélange d'acide tannique et d' hydrogénosulfite de sodium est mélangé avec la formulation du composé d'aluminium soluble dans l'eau contenant de l'acide citrique ou un de ses sels.

5. Procédé selon la revendication 3, dans lequel l'acide tannique est mélangé avec la formulation du composé d'aluminium soluble dans l'eau et avec l'acide citrique ou un de ses sels contenant de l'hydrogénosulfite de sodium.

6. Procédé pour la préparation d'une composition pour traiter des tissus anormaux atteints selon la revendication 3, dans lequel un polyalcool et/ou un glucide sont ou est ajouté(s) tant à l'acide tannique qu'à ladite formulation ou soit à l'un d'entre ledit acide tannique et ladite formulation ou à un mélange de l'acide tannique et de ladite formulation.

7. Procédé selon l'une quelconque des revendications 1 à 6, dans lequel ledit composé d'aluminium soluble dans l'eau est choisi parmi le chlorure d'aluminium, le sulfate d'aluminium, le carbonate d'aluminium, l'acétate d'aluminium, le nitrate d'aluminium, le lactate d'aluminium, le tartrate d'aluminium, le salicylate d'aluminium, le sulfate d'aluminium sodique, le sulfate d'aluminium potassique, le sulfate d'aluminium césique et le sulfate d'aluminium ammonique.

8. Procédé selon l'une quelconque des revendications 1 à 7, dans lequel ledit composé d'aluminium soluble dans l'eau est incorporé dans une proportion de 1 % à 10 % en concentration efficace dans les préparations.

9. Procédé selon l'une quelconque des revendications 1 à 6, dans lequel ledit acide tannique est incorporé dans une proportion de 0,01 % à 2,0 % en concentration efficace dans les préparations.

10. Procédé selon l'une quelconque des revendications 1 à 6, dans lequel ledit acide citrique ou un de ses sels est le citrate de sodium.

11. Procédé selon l'une quelconque des revendications 1 à 6, dans lequel ledit acide citrique ou un de ses sels est incorporé dans une proportion de 10 % à 80 % par rapport à la quantité du composé d'aluminium soluble dans l'eau.

12. Procédé selon l'une quelconque des revendications 1 à 6, dans lequel ledit hydrogénosulfite de sodium est contenu dans une proportion de 50 % à 200 % par rapport à la quantité d'acide tannique.

13. Procédé selon la revendication 12, dans lequel ledit hydrogénosulfite de sodium est incorporé dans une proportion de 70 % à 150 % par rapport à la quantité d'acide tannique.

14. Procédé selon l'une quelconque des revendications 2 ou 6, dans lequel ledit polyalcool et/ou ledit glucide sont ou est choisi(s) parmi le mannitol, le fructose, le xylitol, le glucose, le galactose, le mannose, le lactose et la glycérine.

15. Procédé selon la revendication 14, dans lequel ledit polyalcool et/ou ledit glucide sont ou est incorporé(s) dans une proportion de 3 % à 20 % en concentration efficace dans les préparations.
